# EUROPÄISCHE PATENTSCHRIFT

(11) **EP 0 681 004 B1**
(45) Veröffentlichungstag und Bekanntmachung des Hinweises auf die Patenterteilung: **02.07.1997**
(21) Anmeldenummer: 95106327.0
(22) Anmeldetag: 27.04.1995
(51) Int. Cl.: C09B 33/12, C09B 33/28, C07D 213/71

(54) **Verdoppelte saure Azofarbstoffe mit einer Kupplungskomponente aus der Hydroxypyridonreihe sowie deren Zwischenprodukte**
Double acid azo dyes having a hydroxypyridone-type coupler as well as their intermediate products
Colorants azoiques acides doubles ayant un copulant de type hydroxypyridone ainsi que leurs produits intermédiaires

(30) Priorität: 07.05.1994 DE 4416266
(43) Veröffentlichungstag der Anmeldung: 08.11.1995
(73) Patentinhaber: BASF AKTIENGESELLSCHAFT, 67056 Ludwigshafen (DE)
(72) Erfinder: Lamm, Gunther, Dr., D-67454 Hassloch (DE); Reichelt, Helmut, Dr., D-67435 Neustadt (DE); Wiesenfeldt, Matthias, Dr., D-67125 Dannstadt-Schauernheim (DE)

(56) Entgegenhaltungen:
- WO-A-92/14791
- FR-A- 2 150 406

## Beschreibung

Die vorliegende Erfindung betrifft neue Azofarbstoffe der Formel I 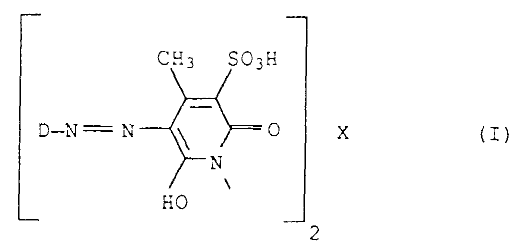 in Form der freien Säure oder ihrer Salze, worin
- D: den Rest einer Diazokomponente und
- X: C₂-C₈-Alkylen, das gegebenenfalls durch ein Sauerstoffatom in Etherfunktion unterbrochen ist, bedeuten,
deren Verwendung zum Färben oder Bedrucken von natürlichen oder synthetischen Substraten, Sulfopyridone als Kupplungskomponenten dieser Farbstoffe sowie ein Verfahren zu ihrer Herstellung.

Aufgabe der vorliegenden Erfindung war es, neue Azofarbstoffe bereitzustellen, die über verdoppelte 3-Hydroxysulfonyl-4-methyl-6-hydroxypyrid-2-one als Kupplungskomponente verfügen und die vorteilhafte anwendungstechnische Eigenschaften aufweisen.

Demgemäß wurden die eingangs näher bezeichneten Azofarbstoffe der Formel I gefunden.

Die neuen Azofarbstoffe der Formel I können entweder in Form der freien Säure oder auch als Salze vorliegen.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z. B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch Hydroxylgruppen substituiert und/oder durch 1 bis 4 Sauerstoffatome in Etherfunktion unterbrochen sein kann.

Alle in der obengenannten Formel auftretenden Alkyl- und Alkylengruppen können sowohl geradkettig als auch verzweigt sein.

Reste X sind z.B. CH₂, (CH₂)₂, (CH₂)₃, (CH₂)₄, (CH₂)₅, (CH₂)₆, (CH₂)₇, (CH₂)₈, CH(CH₃)CH₂, CH(CH₃)CH(CH₃), C₂H₄OC₂H₄, C₃H₆OC₂H₄, C₃H₆OC₃H₆, C₄H₈OC₃H₆ oder C₄H₈OC₄H₈.

Von Bedeutung sind Azofarbstoffe der Formel I, in der D den Rest einer Diazokomponente bedeutet, die sich von einem Anilin, einem Aminonaphthalin oder einem fünfgliedrigen aromatischen heterocyclischen Amin ableitet, das ein bis drei Heteroatome, ausgewählt aus der Gruppe, bestehend aus Stickstoff, Sauerstoff und Schwefel, im heterocyclischen Ring aufweist und durch einen Benzol-, Thiophen-, Pyridin- oder Pyrimidinring anelliert sein kann.

Wichtige Azofarbstoffe der Formel I sind solche, in der D den Rest einer Diazokomponente bedeutet, die sich von einem Anilin, einem Aminonaphthalin oder einem heterocyclischen Amin aus der Pyrrol-, Furan-, Thiophen-, Pyrazol-, Imidazol-, Oxazol-, Isoxazol-, Thiazol-, Isothiazol-, Triazol-, Oxadiazol-, Thiadiazol-, Benzofuran-, Benzthiophen-, Benzimidazol-, Benzoxazol-, Benzthiazol-, Benzisothiazol-, Pyridothiophen-, Pyrimidothiophen-, Thienothiophen- oder Thienothiazolreihe ableitet.

Von besonderer Bedeutung sind Azofarbstoffe der Formel I, in der D den Rest einer Diazokomponente bedeutet, die sich von einem Anilin oder Aminonaphthalin ableitet, wobei Aniline besonders hervorzuheben sind.

Technisch wichtig sind Azofarbstoffe der Formel I, in der D den Rest einer Diazokomponente bedeutet, die sich von einem Aminobenzophenon, einem Aminoazobenzol oder einer Aminobenzoesäure ableitet.

Reste D gehorchen z.B. der Formel 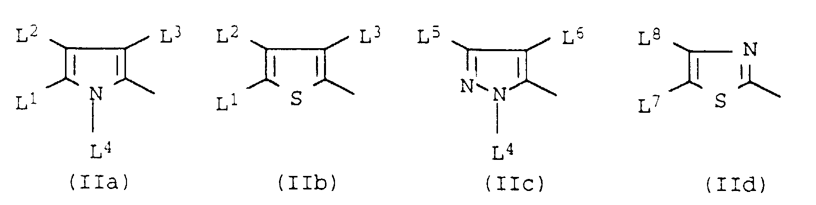 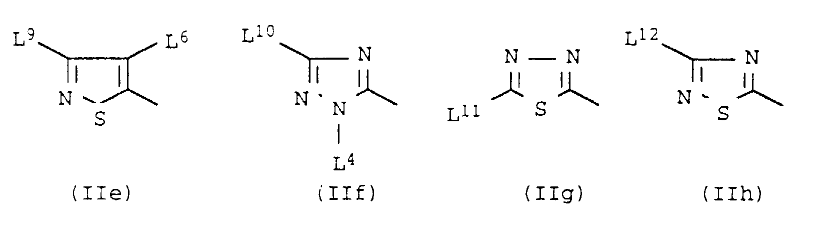 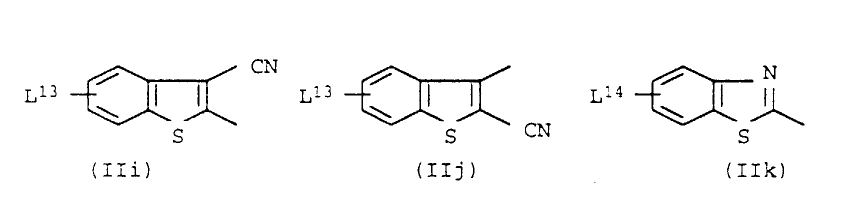 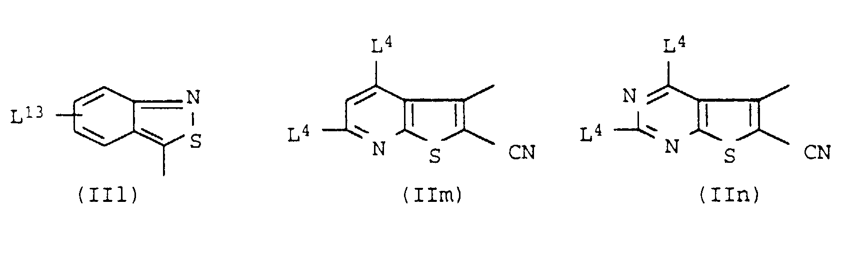 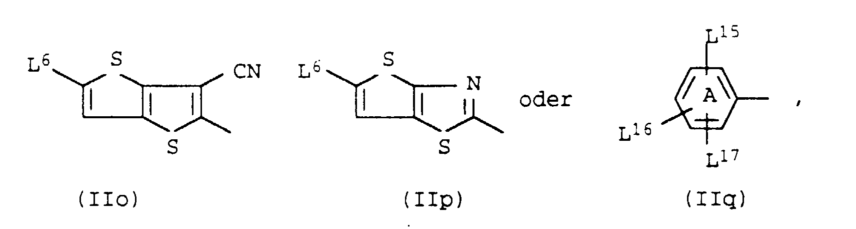 worin
- L¹: Nitro, Cyano, C₁-C₆-Alkanoyl, Benzoyl, C₁-C₆-Alkylsulfonyl, gegebenenfalls substituiertes Phenylsulfonyl oder einen Rest der Formel -CH=T, worin T die Bedeutung von Hydroxyimino, C₁-C₄-Alkoxyimino oder eines Restes einer CH-aciden Verbindung besitzt,
- L²: Wasserstoff, C₁-C₆-Alkyl, Halogen, Hydroxy, Mercapto, gegebenenfalls durch Phenyl oder C₁-C₄-Alkoxy substituiertes C₁-C₆-Alkoxy, gegebenenfalls substituiertes Phenoxy, gegebenenfalls durch Phenyl substituiertes C₁-C₆-Alkylthio, gegebenenfalls substituiertes Phenylthio, C₁-C₆-Alkylsulfonyl oder gegebenenfalls substituiertes Phenylsulfonyl,
- L³: Cyano, C₁-C₄-Alkoxycarbonyl, Carboxyl oder Nitro,
- L⁴: Wasserstoff, C₁-C₆-Alkyl oder Phenyl,
- L⁵: C₁-C₆-Alkyl oder Phenyl,
- L⁶: Wasserstoff, Cyano, C₁-C₄-Alkoxycarbonyl, Carboxyl, C₁-C₆-Alkanoyl, Thiocyanato oder Halogen,
- L⁷: Nitro, Cyano, C₁-C₆-Alkanoyl, Benzoyl, C₁-C₄-Alkoxycarbonyl, Carboxyl, C₁-C₆-Alkylsulfonyl, gegebenenfalls substituiertes Phenylsulfonyl oder einen Rest der Formel -CH=T, worin T die obengenannte Bedeutung besitzt,
- L⁸: Wasserstoff, C₁-C₆-Alkyl, Cyano, Halogen, gegebenenfalls durch Phenyl oder C₁-C₄-Alkoxy substituiertes C₁-C₆-Alkoxy, gegebenenfalls durch Phenyl substituiertes C₁-C₆-Alkylthio, gegebenenfalls substituiertes Phenylthio, C₁-C₆-Alkylsulfonyl, gegebenenfalls substituiertes Phenylsulfonyl, C₁-C₄-Alkoxycarbonyl oder Carboxyl,
- L⁹: Cyano, gegebenenfalls durch Phenyl substituiertes C₁-C₆-Alkyl, gegebenenfalls durch Phenyl substituiertes C₁-C₆-Alkylthio, gegebenenfalls substituiertes Phenyl, Thienyl, C₁-C₄-Alkylthienyl, Pyridyl oder C₁-C₄-Alkylpyridyl,
- L¹⁰: Phenyl oder Pyridyl,
- L¹¹: Trifluormethyl, Nitro, C₁-C₆-Alkyl, Phenyl, gegebenenfalls durch Phenyl substituiertes C₁-C₆-Alkylthio oder C₁-C₆-Dialkylamino,
- L¹²: C₁-C₆-Alkyl, Phenyl, 2-Cyanoethylthio oder 2-(C₁-C₄-Alkoxycarbonyl)ethylthio,
- L¹³: Wasserstoff, Nitro oder Halogen,
- L¹⁴: Wasserstoff, Cyano, C₁-C₄-Alkoxycarbonyl, Carboxyl, Nitro oder Halogen, und
- L¹⁵ und L¹⁶: gleich oder verschieden sind und unabhängig voneinander jeweils Wasserstoff, C₁-C₆-Alkyl, C₁-C₆-Alkoxy oder Halogen und
- L¹⁷: Wasserstoff, C₁-C₆-Alkyl, Trifluormethyl, C₁-C₆-Alkanoyl, gegebenenfalls substituiertes Benzoyl, C₁-C₄-Alkoxycarbonyl, Hydroxysulfonyl, gegebenenfalls substituiertes Phenylsulfonylory, C₁-C₄-Mono- oder Dialkylcarbamoyl, C₁-C₄-Mono- oder oder Dialkylsulfamoyl, C₁-C₄-Alkanoylamino, Hydroxysulfonyl-Carboxylphenylazo, 5-(C₁-C₄-Alkyl)-1,2,4-oxadiazol-3-yl oder 6-Hydroxysulfonyl-7-methylbenzthiazol-2-yl bedeuten, und
der Ring A durch einen Benzolring oder Hydroxysulfonylbenzolring anelliert sein kann.

Alle in den obengenannten Formeln auftretenden Alkyl- oder Alkylengruppen können sowohl geradkettig als auch verzweigt sein.

Wenn in den obengenannten Formeln der Azofarbstoffe substituierte Phenylgruppen auftreten, können als Substituenten z.B. C₁-C₄-Alkyl, Chlor, Brom, Nitro oder C₁-C₄-Alkoxy in Betracht kommen. Die Phenylreste weisen dabei in der Regel 1 bis 3 Substituenten auf.

Wenn in den obengenannten Formeln der Azofarbstoffe substituierte Alkylgruppen auftreten, weisen diese in der Regel 1 oder 2 Substituenten auf.

Reste L², L⁴, L⁵, L⁸, L⁹, L¹¹, L¹², L¹⁵, L¹⁶, L¹⁷ sind z.B. Methyl, Ethyl, Propyl, Isopropyl, Butyl, Isobutyl, sec-Butyl, Pentyl, Isopentyl, Neopentyl, tert-Pentyl, Hexyl oder 2-Methylpentyl.

Reste L⁹ sind weiterhin z.B. Benzyl oder 1- oder 2-Phenylethyl.

Reste L², L⁸, L⁹ und L¹¹ sind weiterhin z.B. Methylthio, Ethylthio, Propylthio, Isopropylthio, Butylthio, Isobutylthio, Pentylthio, Hexylthio, Benzylthio oder 1- oder 2-Phenylethylthio.

Reste L² und L⁸ sind weiterhin z.B. Phenylthio, 2-Methylphenylthio, 2-Methoxyphenylthio oder 2-Chlorphenylthio.

Reste L², L⁸, L¹⁵ und L¹⁶ sind weiterhin z.B. Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy oder 2-Methylpentyloxy.

Reste L², L⁸, L¹³, L¹⁴, L¹⁵ und L¹⁶ sind weiterhin z.B. Fluor, Chlor oder Brom.

Reste L⁷ sind, wie weiterhin auch Reste L¹, L² und L^{8,} z.B. Methylsulfonyl, Ethylsulfonyl, Propylsulfonyl, Isopropylsulfonyl, Butylsulfonyl, Isobutylsulfonyl sec-Butylsulfonyl, Pentylsulfonyl, Isopentylsulfonyl, Neopentylsulfonyl, Hexylsulfonyl, Phenylsulfonyl, 2-Methylphenylsulfonyl, 2-Methoxyphenylsulfonyl oder 2-Chlorphenylsulfonyl.

Reste L³ sind, wie weiterhin auch Reste L⁶, L⁷, L⁸, L¹⁴ und L¹⁷, z.B. Methoxycarbonyl, Ethoxycarbonyl, Propoxycarbonyl, Isopropoxycarbonyl, Butoxycarbonyl, Isobutoxycarbonyl oder sec-Butoxycarbonyl.

Reste L² und L⁸ sind weiterhin z.B. 2-Methoxyethoxy, 2-Ethoxyethoxy, 2- oder 3-Methoxypropoxy, 2- oder 3-Ethoxypropoxy, 2- oder 4-Methoxybutoxy, 2- oder 4-Ethoxybutoxy, 5-Methoxypentyloxy, 5-Ethoxypentyloxy, 6-Methoxyhexyloxy, 6-Ethoxyhexyloxy, Benzyloxy oder 1- oder 2-Phenylethoxy.

Reste L¹¹ sind weiterhin z.B. Dimethylamino, Diethylamino, Dipropylamino, Diisopropylamino, Dibutylamino, Dipentylamino, Dihexylamino oder N-Methyl-N-ethylamino.

Reste L¹² sind weiterhin z.B. 2-Methoxycarbonylethylthio oder 2-Ethoxycarbonylethylthio.

Reste L⁹ sind weiterhin z.B. Phenyl, 2-, 3- oder 4-Methylphenyl, 2,4-Dimethylphenyl, 2-, 3- oder 4-Methoxyphenyl, 2-, 3- oder 4-Chlorphenyl, 2-, 3- oder 4-Methoxyphenyl, 2- oder 3-Methylthienyl oder 2-, 3- oder 4-Methylpyridyl.

Reste L¹, L⁶, L⁷ und L¹⁷ sind weiterhin z.B. Formyl, Acetyl, Propionyl, Butyryl, Pentanoyl oder Hexanoyl.

Reste L¹⁷ sind weiterhin z.B. Benzoyl, 2-, 3- oder 4-Methylbenzoyl, 2-, 3- oder 4-Ethylbenzoyl, 2-, 3- oder 4-Propylbenzoyl, 2-, 3- oder 4-Isopropylbenzoyl, 2,3-, 2,4-, 2,5-, 2,6-, 3,4- oder 3,5-Dimethylbenzoyl, 2-Methyl-4-methoxybenzoyl, 2-, 3- oder 4-Methoxybenzoyl, 2-, 3- oder 4-Ethoxybenzoyl, Phenylsulfonyloxy, 2-, 3- oder 4-Methylphenylsulfonyloxy, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl, Formylamino, Acetylamino, Propionylamino, Butyrylamino, Isobutyrylamino, 4-Hydroxysulfonylphenylazo, 4-Carboxylphenylazo, 5-Methyl-1,2,4-oxadiazol-3-yl oder 5-Ethyl-1,2,4-oxadiazol-3-yl.

Wenn L¹ oder L⁷ für den Rest -CH=T stehen, worin T sich von einer CH-aciden Verbindung H₂T ableitet, können als CH-acide Verbindungen H₂T z.B. Verbindungen der Formel 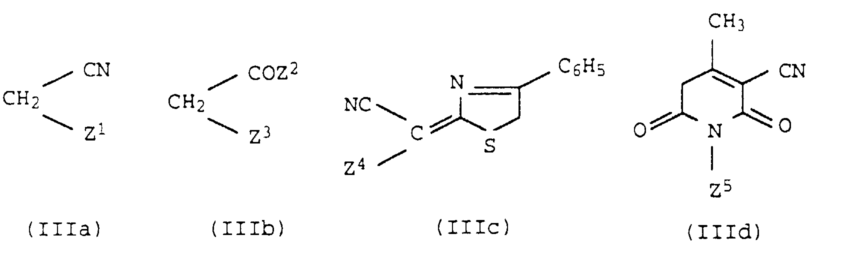 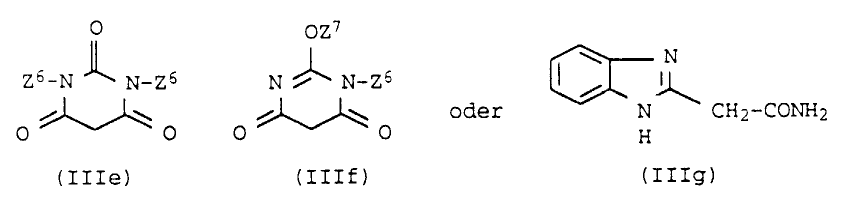 in Betracht kommen, wobei
- Z¹: Cyano, Nitro, C₁-C₄-Alkanoyl, gegebenenfalls substituiertes Benzoyl, C₁-C₄-Alkylsulfonyl, gegebenenfalls substituiertes Phenylsulfonyl, Carboxyl, C₁-C₄-Alkoxycarbonyl, C₃-C₄-Alkenyloxycarbonyl, Phenoxycarbonyl, Carbamoyl, C₁-C₄-Mono- oder Dialkylcarbamoyl, gegebenenfalls substituiertes Phenylcarbamoyl, gegebenenfalls substituiertes Phenyl, Benzthiazol-2-yl, Benzimidazol-2-yl, 5-Phenyl-1,3,4-thiadiazol-2-yl oder 2-Hydroxychinoxalin-3-yl,
- Z²: C₁-C₄-Alkyl, C₁-C₄-Alkoxy oder C₃-C₄-Alkenyloxy,
- Z³: C₁-C₄-Alkoxycarbonyl, C₃-C₄-Alkenyloxycarbonyl, Phenylcarbamoyl oder Benzimidazol-2-yl,
- Z⁴: Cyano, C₁-C₄-Alkoxycarbonyl oder C₃-C₄-Alkenyloxycarbonyl,
- Z⁵: Wasserstoff oder C₁-C₆-Alkyl,
- Z⁶: Wasserstoff, C₁-C₄-Alkyl oder Phenyl und
- Z⁷: C₁-C₄-Alkyl bedeuten.

Dabei ist der Rest, der sich von Verbindungen der Formel IIIa, IIIb oder IIIc ableitet, worin Z¹ Cyano, C₁-C₄-Alkanoyl, C₁-C₄-Alkoxycarbonyl oder C₃-C₄-Alkenyloxycarbonyl, Z² C₁-C₄-Alkyl, C₁-C₄-Alkoxy oder C₃-C₄-Alkenyloxy, Z³ C₁-C₄-Alkoxycarbonyl oder C₃-C₄-Alkenyloxycarbonyl und Z⁴ Cyano bedeuten, hervorzuheben.

Besonders hervorzuheben ist dabei der Rest der sich von Verbindungen der Formel IIIa, IIIb oder IIIc ableitet, worin Z¹ Cyano, C₁-C₄-Alkoxycarbonyl oder C₃-C₄-Alkenyloxycarbonyl, Z² C₁-C₄-Alkoxy oder C₂-C₄-Alkenyloxy, Z³ C₁-C₄-Alkoxycarbonyl oder C₃-C₄-Alkenyloxycarbonyl und Z⁴ Cyano bedeuten.

Bevorzugt sind Azofarbstoffe der Formel Ia 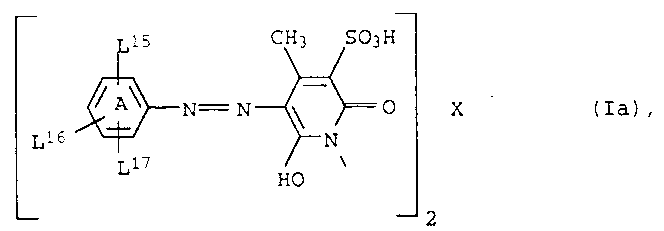 in der L¹⁵, L¹⁶, L¹⁷, X und der Ring A jeweils die obengenannte Bedeutung besitzen.

Besonders bevorzugt sind Azofarbstoffe der Formel Ib 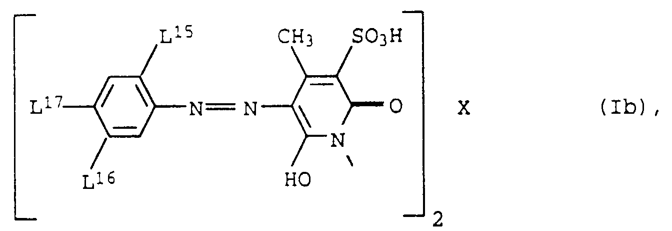 in der
X die obengenannte Bedeutung besitzt und L¹⁵ und L¹⁶ jeweils Wasserstoff und L¹⁷ gegebenenfalls substituiertes Benzoyl oder L¹⁵ und L¹⁶ unabhängig voneinander jeweils Wasserstoff, C₁-C₄-Alkyl oder C₁-C₄-Alkoxy und L¹⁷ Hydroxysulfonylphenylazo oder Carboxylphenylazo bedeuten.

Besonders bevorzugt sind weiterhin Azofarbstoffe der Formel Ic 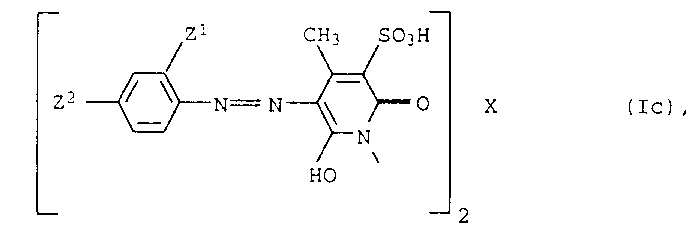 in der einer der beiden Reste Z¹ und Z² Wasserstoff und der andere C₁-C₄-Alkoxycarbonyl bedeuten und X die obengenannte Bedeutung besitzt.

Die erfindungsgemäßen Azofarbstoffe der Formel I können nach an sich bekannten Methoden erhalten werden. Beispielsweise kann man ein Amin der Formel IV

D―NH₂ (IV),

in der D die obengenannte Bedeutung besitzt, diazotieren und das resultierende Diazoniumsalz mit einem Sulfopyridon der Formel V 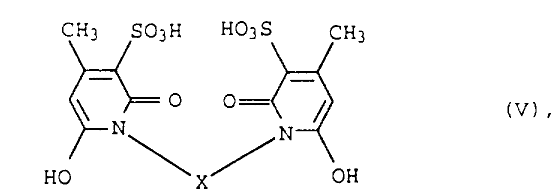 in der X die obengenannte Bedeutung besitzt, kuppeln.

Ein weiterer Gegenstand der vorliegenden Anmeldung sind Sulfopyridone der Formel V 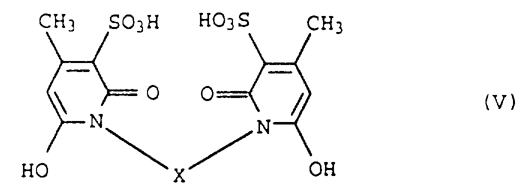 in Form der freien Säure oder ihrer Salze, worin X C₂-C₈-Alkylen, das gegebenenfalls durch ein Sauerstoffatom in Etherfunktion unterbrochen ist, bedeutet.

Es wurde weiterhin gefunden, daß die Sulfopyridone der Formel V vorteilhaft erhalten werden, wenn man ein Cyanopyridon der Formel VI 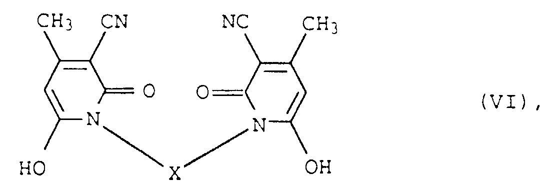 in der X die obengenannte Bedeutung besitzt, in konzentrierter Schwefelsäure bei einer Temperatur von 80 bis 130°C behandelt.

Bevorzugt wird das neue Verfahren bei einer Temperatur von 90 bis 125°C durchgeführt.

Unter konzentrierter Schwefelsäure wird erfindungsgemäß 96 bis 98 gew.-%ige Schwefelsäure verstanden. Je Gewichtsteil Cyanopyridon VI kommen dabei in der Regel 2 bis 2,5 Gewichtsteile konzentrierte Schwefelsäure zur Anwendung.

Das neue Verfahren wird in der Regel so vorgenommen, daß man konzentrierte Schwefelsäure vorlegt und dazu bei Raumtemperatur das Cyanopyridon VI gibt. Dabei erfolgt eine Temperaturerhöhung, und man läßt die Temperatur auf ca. 60 bis 70°C ansteigen. Danach erfolgt eine Nachrührphase bei der erfindungsgemäßen Temperatur. Nach 5 bis 8 Stunden ist die Umsetzung in der Regel beendet, und man läßt dann abkühlen. Das Reaktionsgemisch kann dann in eine Eis-Wasser-Mischung gegeben und neutralisiert werden. Die so erhaltene Lösung kann direkt zur Herstellung der Azofarbstoffe der Formel I verwendet werden.

Es kann von Vorteil sein, in Gegenwart von geringen Mengen an Schwefeltrioxid zu arbeiten. In diesem Fall stellt das Reaktionsmedium z.B. 2 bis 5 gew.-%iges Oleum dar.

Die erfindungsgemäßen Azofarbstoffe der Formel I eignen sich in vorteilhafter Weise zum Färben von natürlichen oder synthetischen Substraten, beispielsweise von Wolle, Leder oder Polyamid. Man erhält Färbungen mit guten Gebrauchsechtheiten.

Die folgenden Beispiele sollen die Erfindung näher erläutern.

### Beispiel 1

163 g des Cyanopyridons der Formel 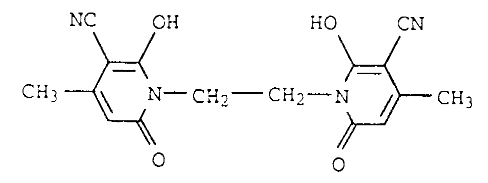 wurden in ein Gemisch von 40 g Oleum (24 gew.-%ig) und 380 g Schwefelsäure (100 gew.-%ig) eingetragen. Dabei ließ man die Temperatur von 15°C zu Beginn der Zugabe auf maximal 70°C ansteigen. Man rührte 2 h bei 70 bis 80°C und erwärmte dann auf 90°C, wobei CO₂ abgespalten wurde. Dann hob man die Reaktionstemperatur innerhalb von ca. 4 h so auf 130 bis 135°C an, daß sich die CO₂-Abspaltung gut beherrschen ließ. Anschließend wurde noch 6 h bei 130 bis 135°C nachgerührt.

Die nach dem Abkühlen erhaltene Schmelze wurde auf ca. 1000 g Eis und 500 ml Wasser ausgerührt und die Fällung dabei unter Kühlung mit Natronlauge bei < 40°C auf einen pH-Wert von 7 gestellt. Man erhielt eine klare Lösung (die beim Stehenlassen Natriumsulfat abscheiden kann) des Sulfopyridons der Formel

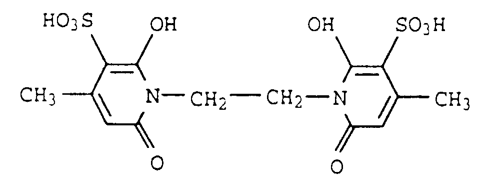

### Beispiel 2

340 g des Cyanopyridons der Formel 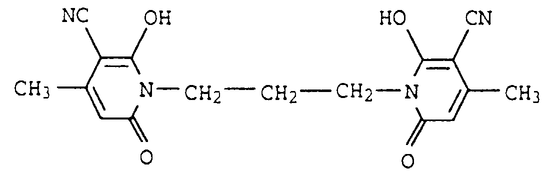 wurden in ein Gemisch aus 760 g Schwefelsäure (100 gew.-%ig) und 80 g Oleum (24 gew.-%ig) analog Beispiel 1 eingetragen und umgesetzt. Man erhielt ca. 0,97 mol des Sulfopyridons der Formel 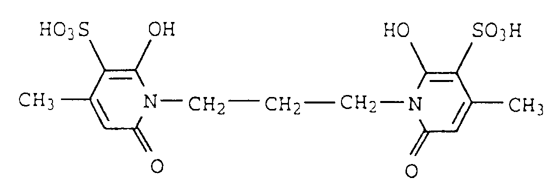 in Form einer wäßrigen Lösung.

### Beispiel 3

30 g 4-Amino-4-methylbenzophenon-hydrochlorid wurden in 55 ml 17 gew.-%iger Salzsäure verrührt. Dann kühlte man das Gemisch auf 0°C ab, gab ca. 40 g Eis und gleichzeitig 20 ml 23 gew.-%ige wäßrige Natriumnitritlösung so hinzu, daß die Temperatur der Diazotierung nicht über 10°C anstieg. Anschließend rührte man 1 h nach und kühlte dabei mit Eis auf 0 bis 5°C ab. Nach Zerstören von überschüssiger salpetriger Säure hob man den pH-Wert der erhaltenen Diazoniumsalzlösung mit etwas Natriumacetat und Natronlauge bei < 0°C auf 5 bis 6 an. Dann ließ man 14,85 g des Sulfopyridons der Formel 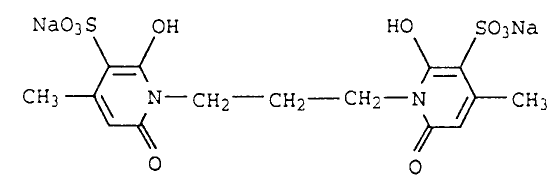 als wäßrige Lösung innerhalb von 20 min zulaufen und hielt den pH-Wert dabei durch Zugabe von Natronlauge im Bereich von 4 bis 7,5.

Der Farbstoff der Formel 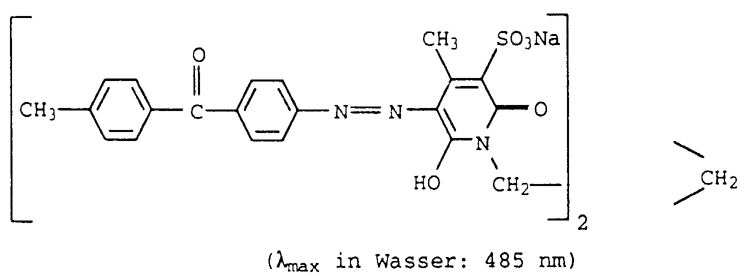 fiel kristallin aus und wurde bei einem pH-Wert von 7 als Dinatriumsalz durch Aussalzen isoliert. Nach dem Trocknen erhielt man 45 g eines gelben Pulvers, das sich in Wasser mit gelber Farbe löst. Färbt man 100 g eines nachgegerbten Chromrindsleders mit 0,8 g dieses Farbstoffes nach den üblichen Färbeverfahren, so erhält man eine farbstarke, zitronengelbe Oberflächenfärbung mit klarer Nuance.

Polycaprolactamgewebe wird nach üblichen Färbeverfahren in klaren, kräftigen, zitronengelben Nuancen angefärbt, Wolle in kräftigen neutralgelben Tönen.

### Beispiel 4

10 g 4-Aminobenzoesäureethylester wurden analog Beispiel 3 diazotiert. Man zerstörte überschüssige salpetrige Säure, hob den pH-Wert der Diazoniumsalzlösung mit wenig Natriumacetat und Natronlauge bei 0°C auf 4,5 bis 6,5, ließ dann 14,85 g des in Beispiel 2 genannten Sulfopyridons als wäßrige Lösung zulaufen und hielt den pH-Wert der Kupplung dabei im oben angegebenen Bereich. Man erhielt 25 g des Farbstoffes der Formel 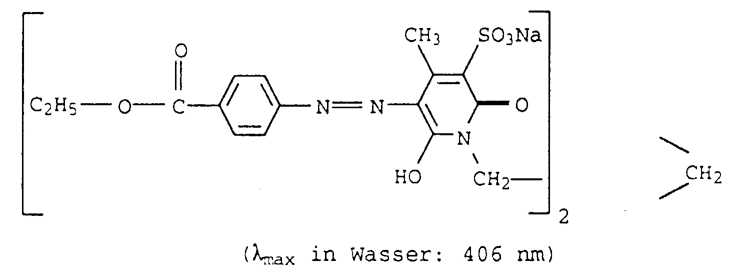 Isolierung bei pH ca. 6,5 und Trocknung erfolgten wie üblich. Der Farbstoff löst sich in Wasser mit gelber Farbe und färbt Leder, Polycaprolactam und Wolle in kräftiger, zitronengelber bis goldgelber Nuance. Die Färbungen haben gute Licht- und Naßechtheiten.

### Beispiel 5

16,7 g 4-Aminoazobenzol-4-sulfonsäure wurden in 100 ml Wasser mit Natronlauge bei einem pH-Wert von 8 bis 9 heiß gelöst. Dann gab man 20 ml 23 gew.-%ige wäßrige Natriumnitritlösung hinzu und ließ unter Rühren erkalten. Nach Abkühlen mit Eis auf 0°C säuerte man mit 20 ml konz. Salzsäure an. Die erhaltene Suspension wurde 4 h bei 0 bis 5°C gerührt. Danach zerstörte man überschüssige salpetrige Säure, hob den pH-Wert der Suspension mit wenig Natriumacetat und Natronlauge auf 4,5 bis 5,5 an und ließ analog Beispiel 4 eine wäßrige Lösung von 14,5 g des Natriumsalzes der in Beispiel 1 beschriebenen Kupplungskomponente zulaufen. Anschließend wurde das Reaktionsgemisch mit Natronlauge auf einen pH-Wert von 5,5 gestellt. Der entstandene Farbstoff wurde mit Natriumchlorid ausgefällt, die Fällung wie üblich isoliert und getrocknet. Man erhielt 31 g eines orangeroten Pulvers der Formel 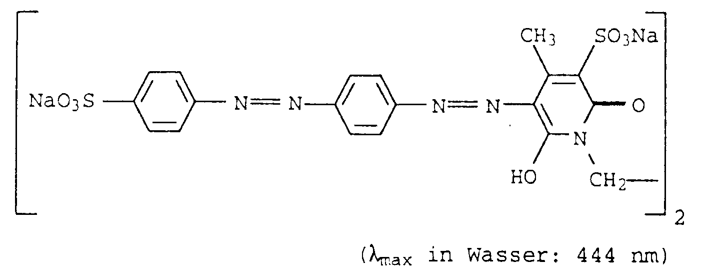 das sich in Wasser mit goldgelbem Farbton löst. Der Farbstoff färbt Wolle, Polycaprolactam und Leder in kräftigem orangefarbenem Ton mit guten Echtheiten an.

Analog den Beispielen 1 bis 5 werden die in den folgenden Tabellen 1 und 2 beschriebenen Farbstoffe erhalten.

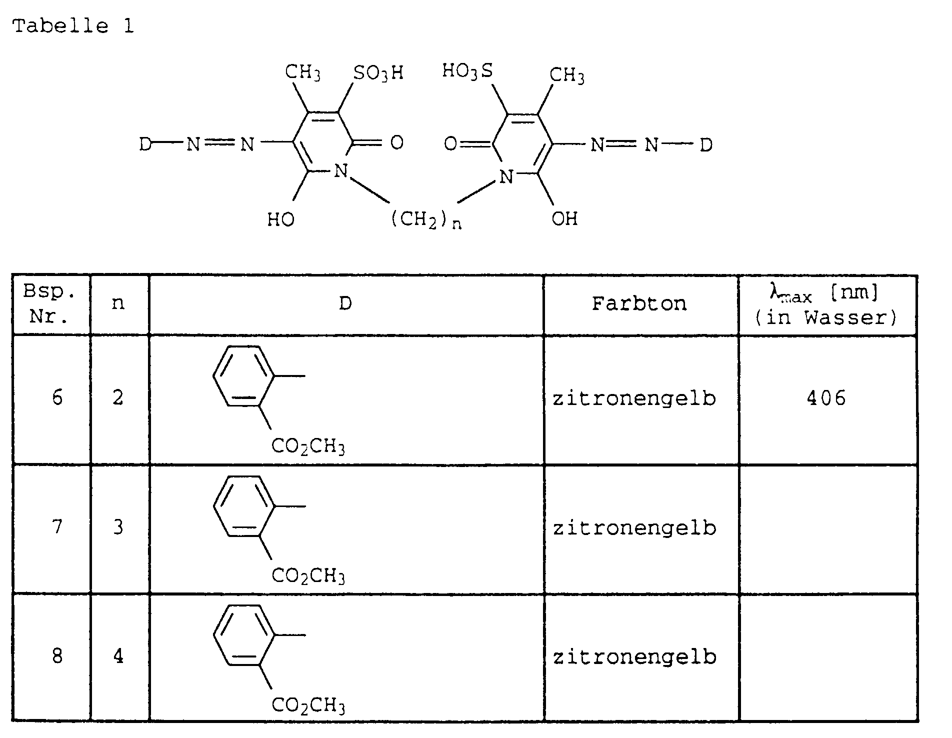

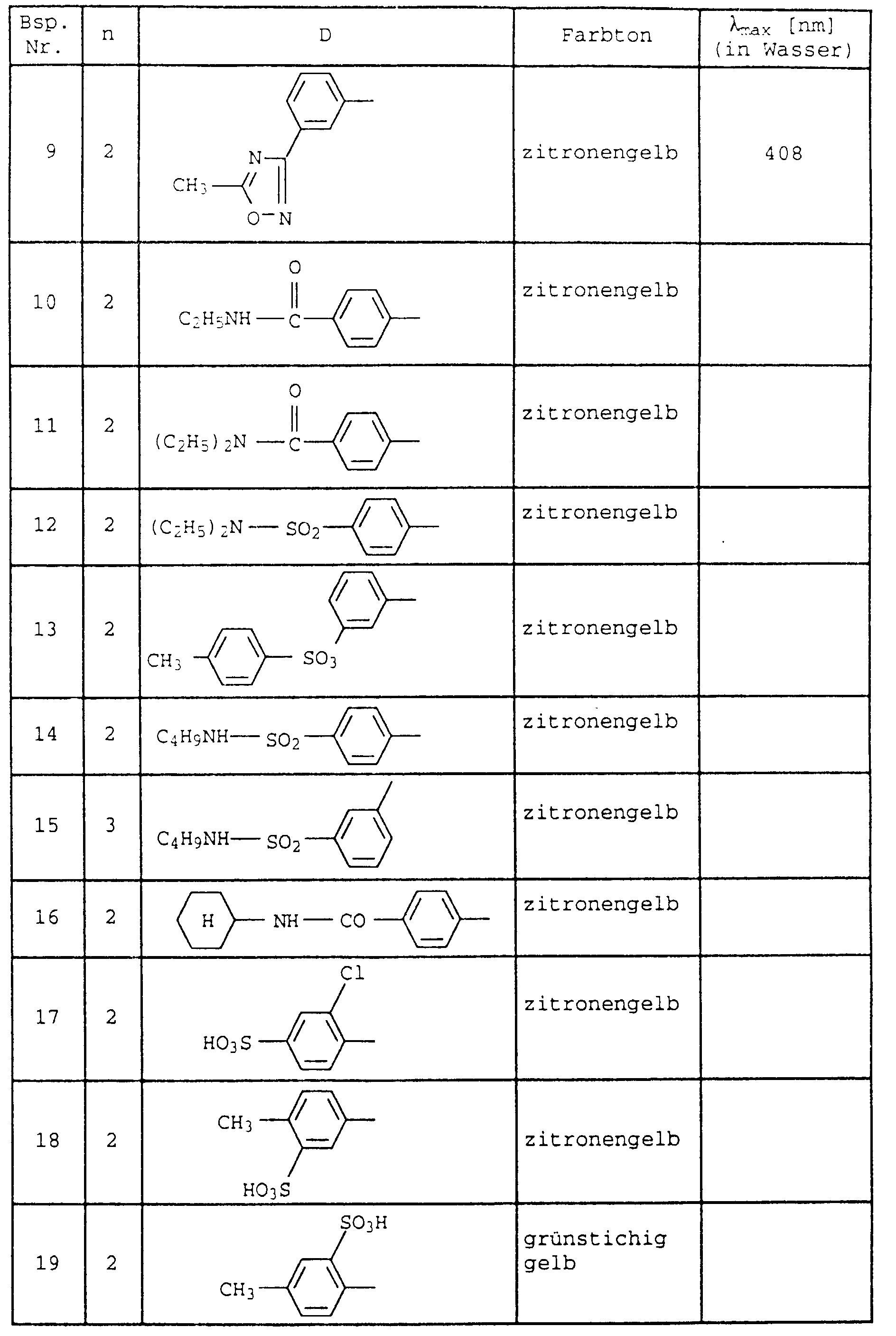

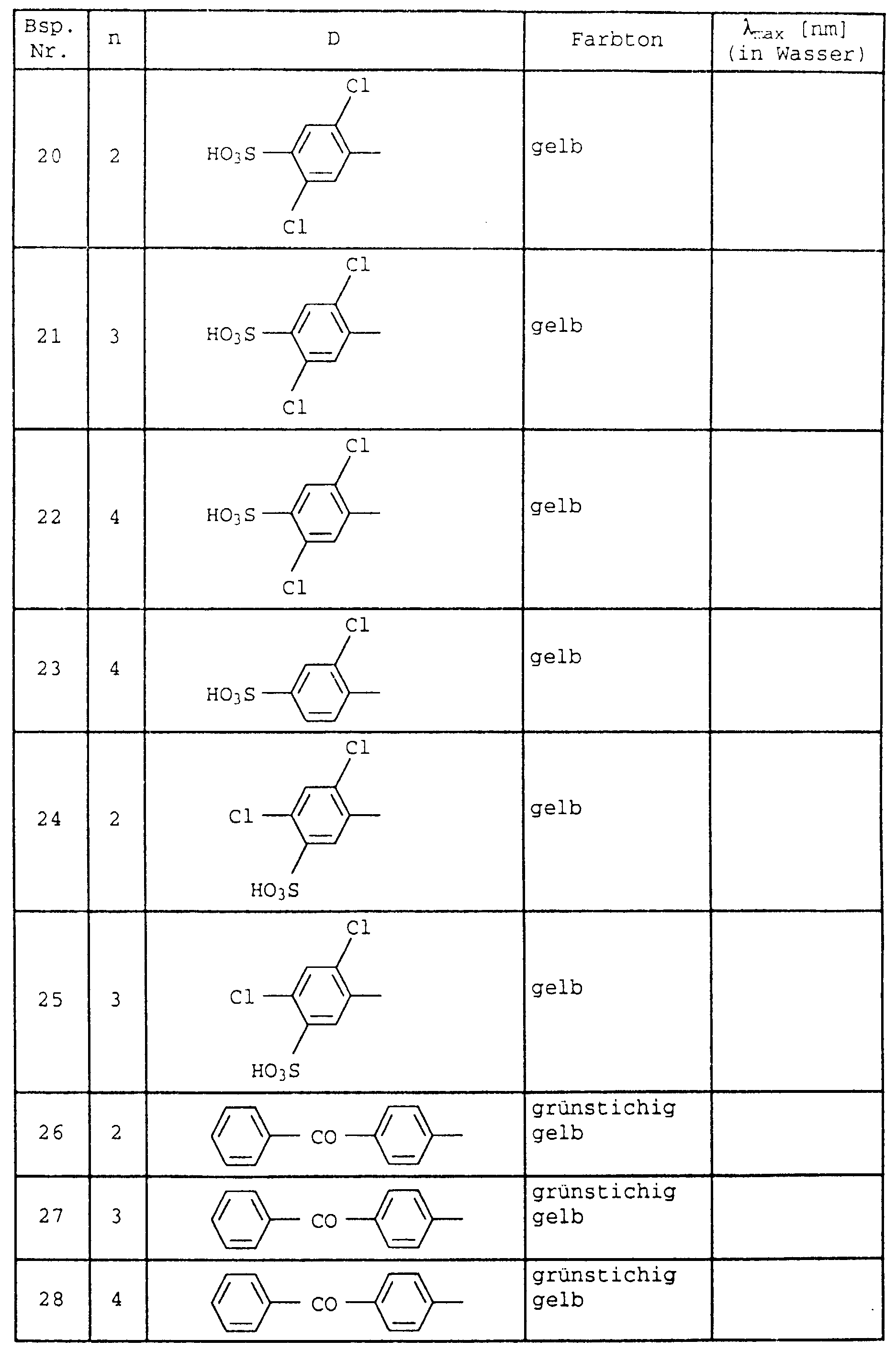

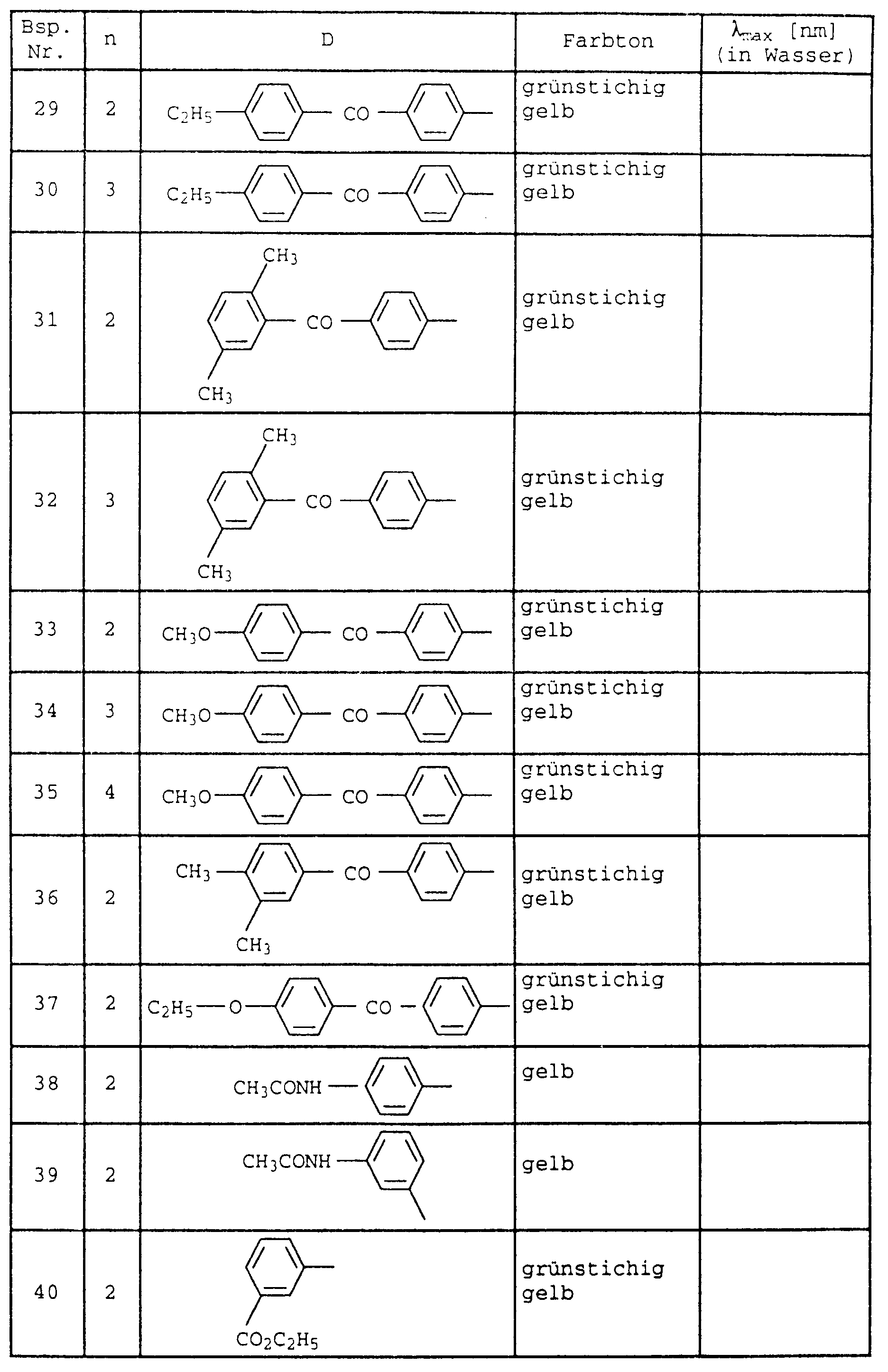

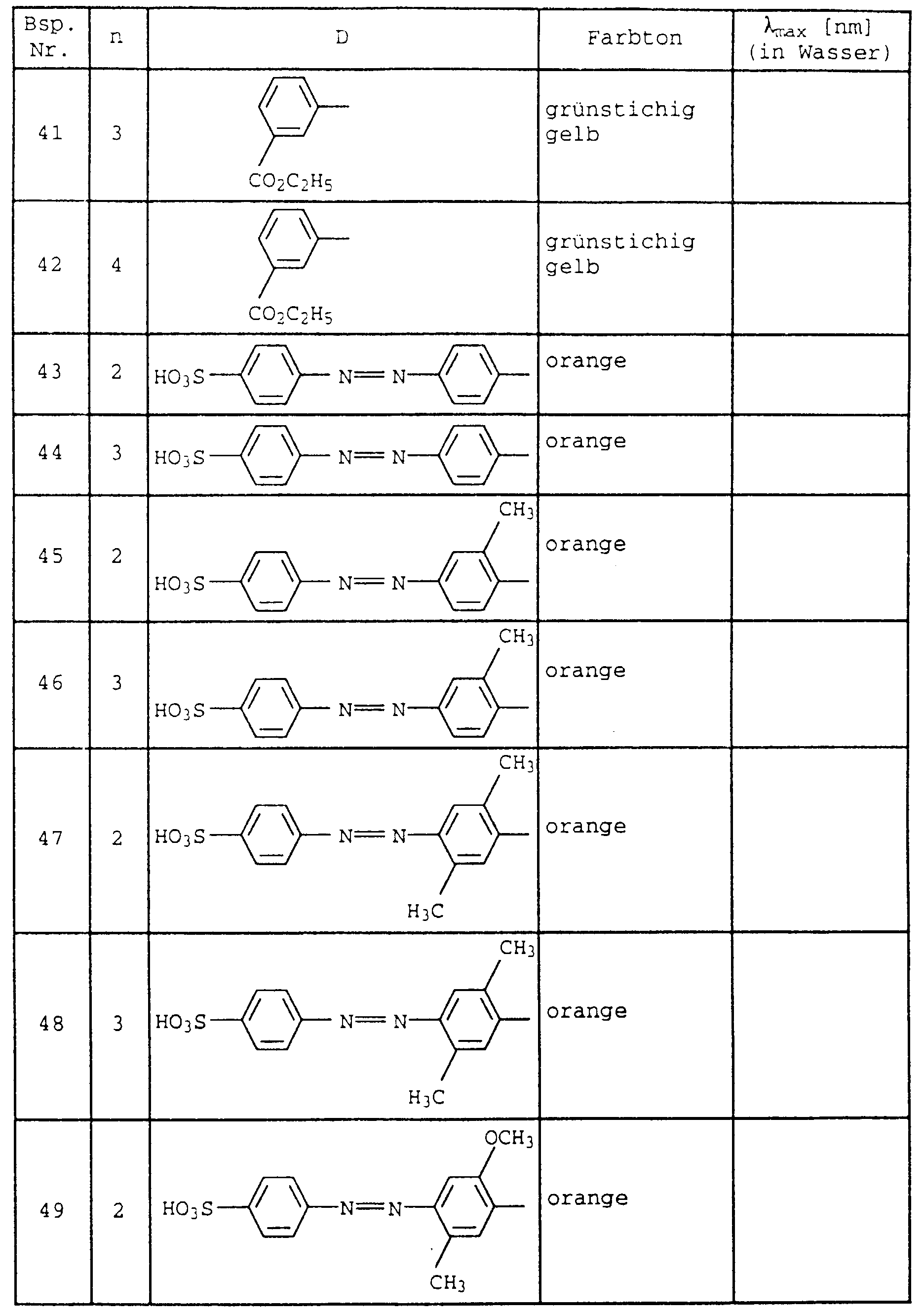

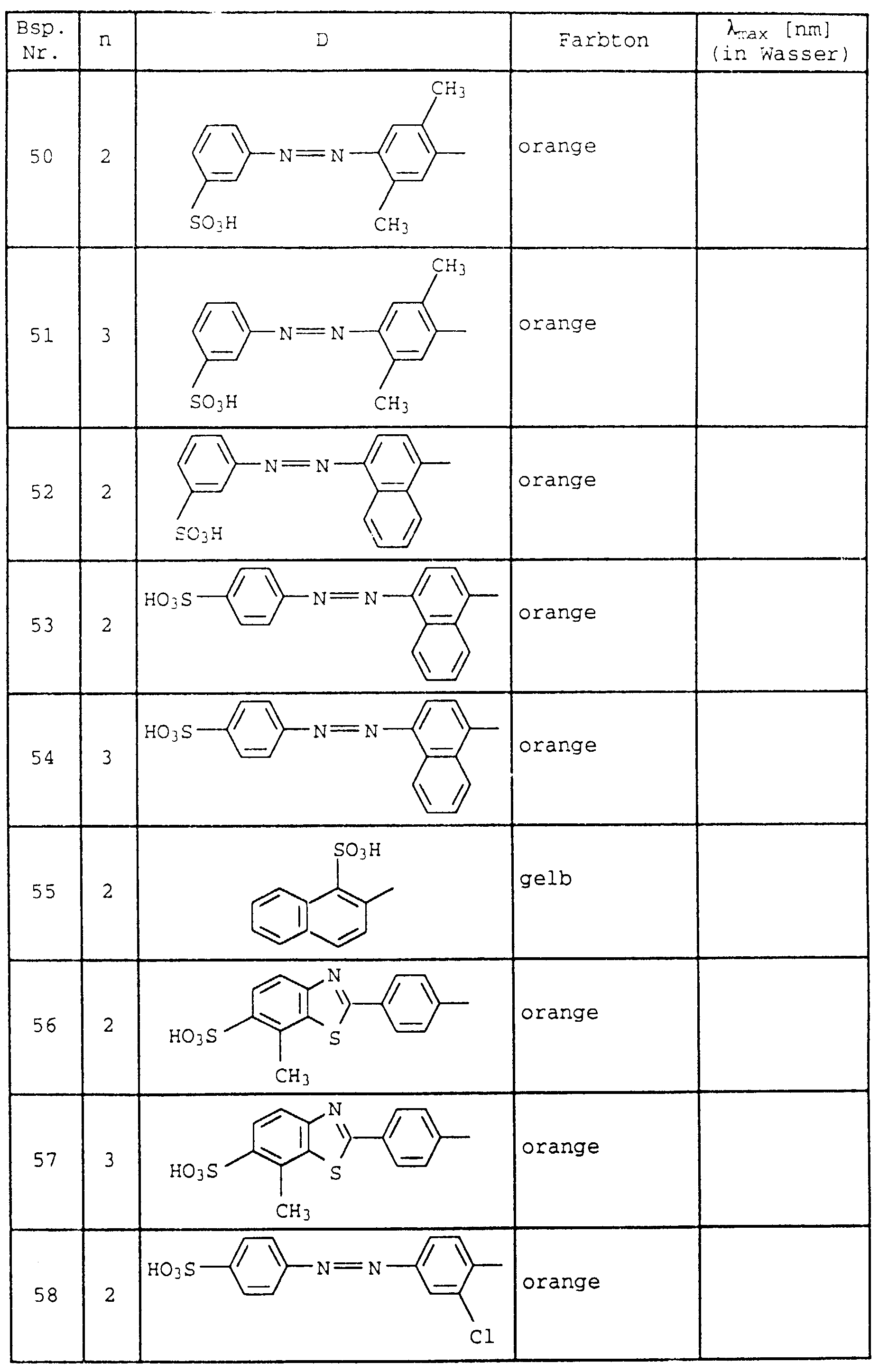

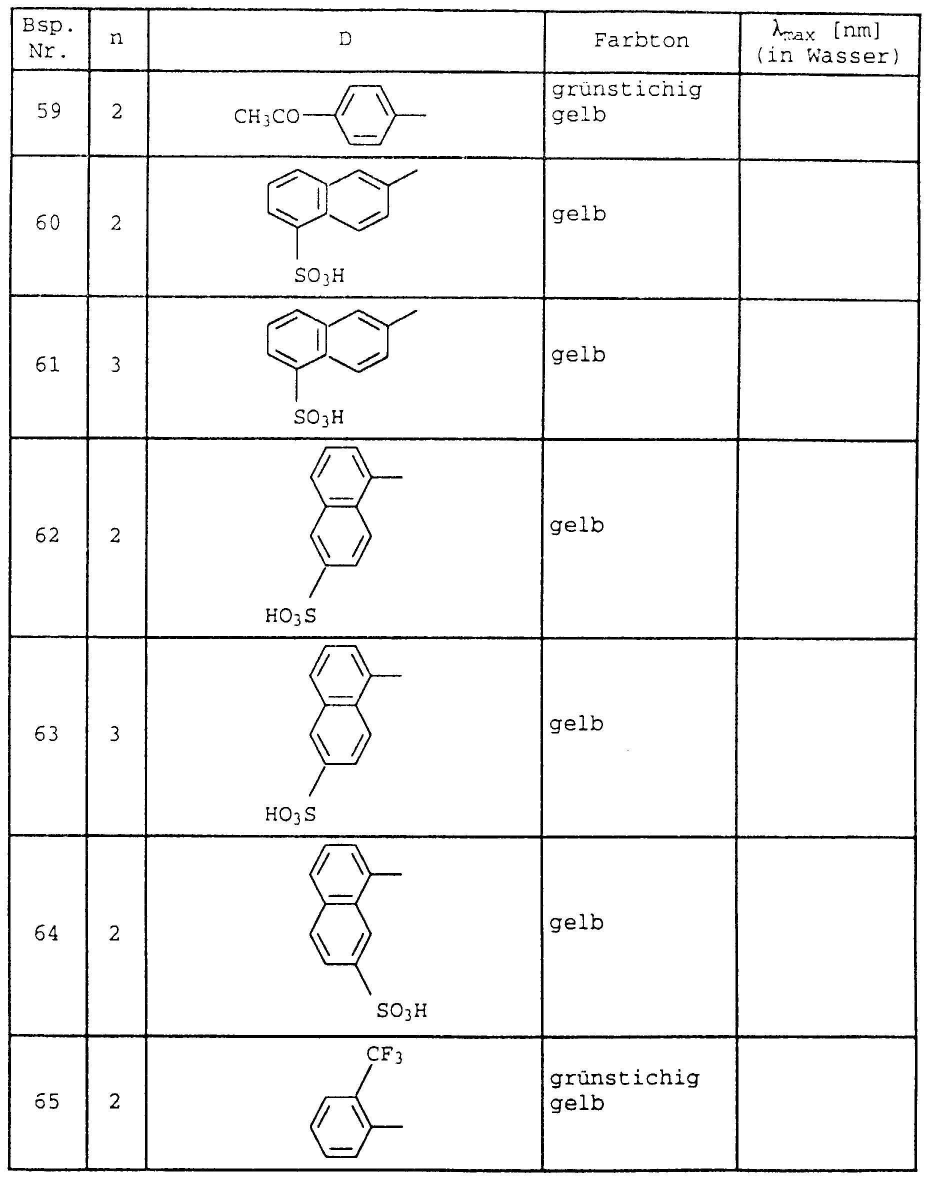

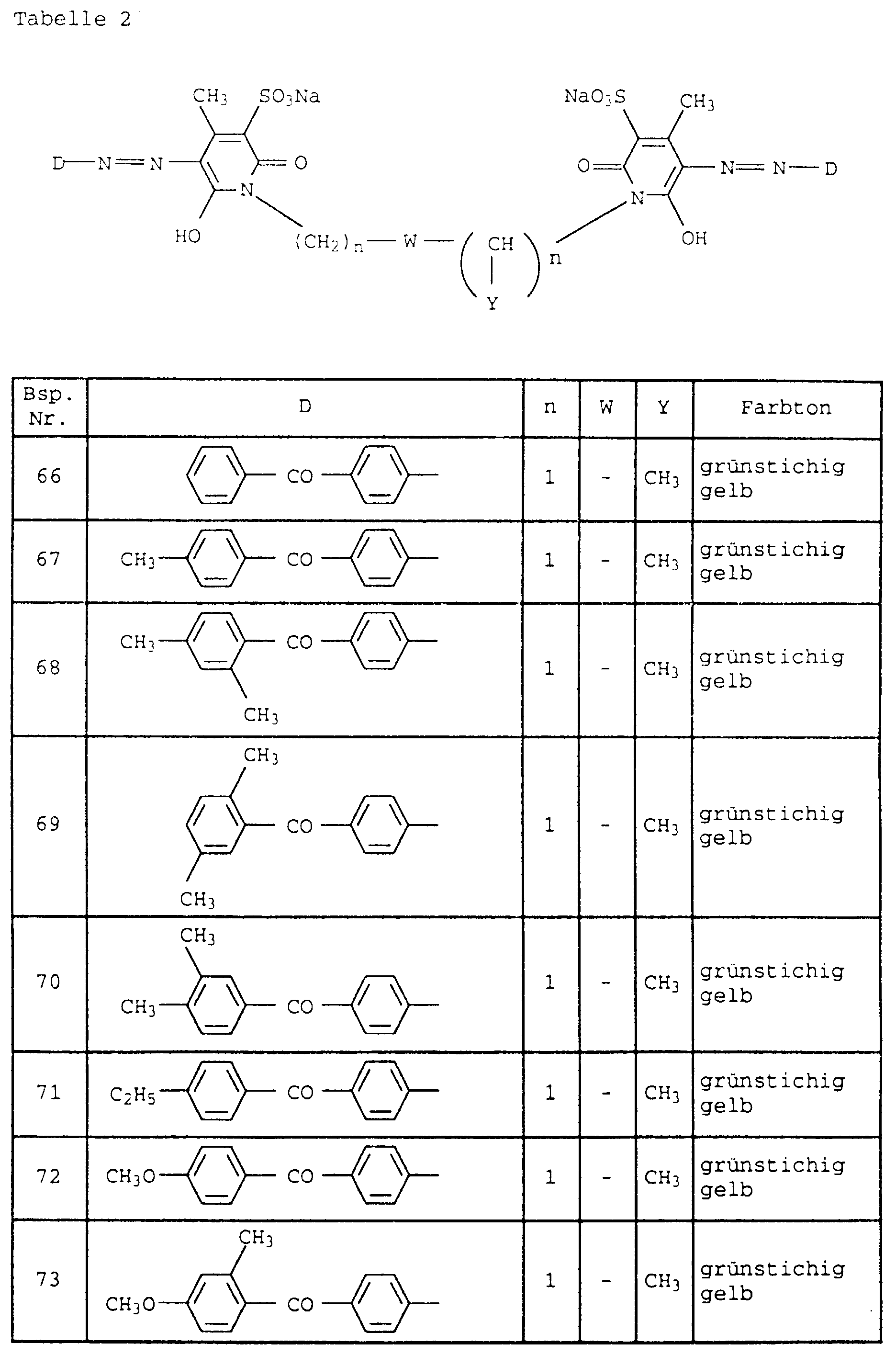

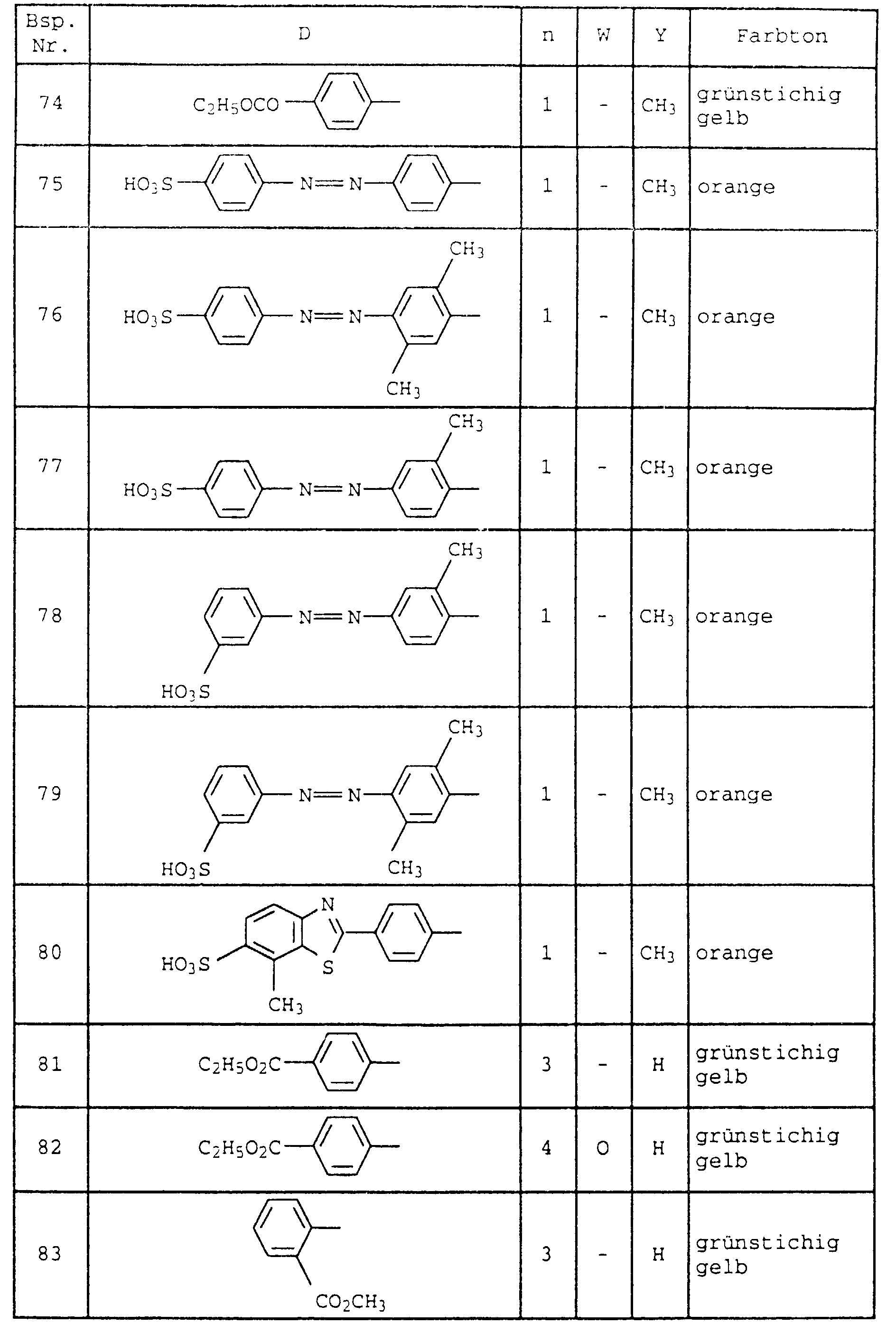

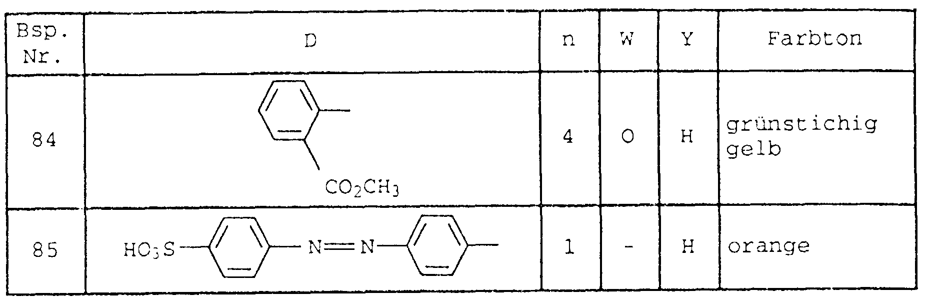

## Patentansprüche (Patentansprüche für folgende(n) Vertragsstaat(en): CH, DE, FR, GB, IT, LI)

1. Azofarbstoffe der Formel I 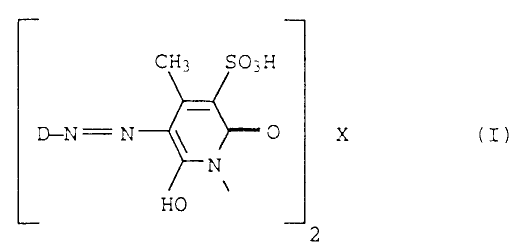 in Form der freien Säure oder ihrer Salze, worin
D den Rest einer Diazokomponente und
X C₂-C₈-Alkylen, das gegebenenfalls durch ein Sauerstoffatom in Etherfunktion unterbrochen ist, bedeuten.

2. Azofarbstoffe nach Anspruch 1, dadurch gekennzeichnet, daß D den Rest einer Diazokomponente bedeutet, die sich von einem Anilin, einem Aminonaphthalin oder einem fünfgliedrigen aromatischen heterocyclischen Amin ableitet, das ein bis drei Heteroatome, ausgewählt aus der Gruppe, bestehend aus Stickstoff, Sauerstoff und Schwefel, im heterocyclischen Ring aufweist und durch einen Benzol-, Thiophen-, Pyridin- oder Pyrimidinring anelliert sein kann.

3. Azofarbstoffe nach Anspruch 1, dadurch gekennzeichnet, daß D den Rest einer Diazokomponente bedeutet, die sich von einem Anilin, einem Aminonaphthalin oder einem heterocyclischen Amin aus der Pyrrol-, Furan-, Thiophen-, Pyrazol-, Imidazol-, Oxazol-, Isoxazol-, Thiazol-, Isothiazol-, Triazol-, Oxadiazol-, Thiadiazol-, Benzofuran-, Benzthiophen-, Benzimidazol-, Benzoxazol, Benzthiazol-, Benzisothiazol-, Pyridothiophen-, Pyrimidothiophen-, Thienothiophen- oder Thienothiazolreihe ableitet.

4. Azofarbstoffe nach Anspruch 1, dadurch gekennzeichnet, daß D den Rest einer Diazokomponente bedeutet, die sich von einem Anilin oder einem Aminonaphthalin ableitet.

5. Azofarbstoffe nach Anspruch 1, dadurch gekennzeichnet, daß D den Rest einer Diazokomponente bedeutet, die sich von einem Anilin ableitet.

6. Azofarbstoffe nach Anspruch 1, dadurch gekennzeichnet, daß D den Rest einer Diazokomponente bedeutet, die sich von einem Aminobenzophenon, einem Aminoazobenzol oder einer Aminobenzoesäure ableitet.

7. Verwendung der Azofarbstoffe gemäß Anspruch 1 zum Färben oder Bedrucken von natürlichen oder synthetischen Substraten.

8. Sulfopyridone der Formel V 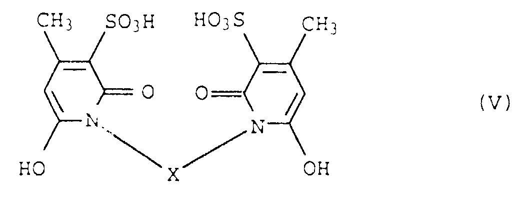 in Form der freien Säure oder ihrer Salze, worin X C₂-C₈-Alkylen, das gegebenenfalls durch ein Sauerstoffatom in Etherfunktion unterbrochen ist, bedeutet.

9. Verfahren zur Herstellung der Sulfopyridone gemäß Anspruch 8, dadurch gekennzeichnet, daß man ein Cyanopyridon der Formel VI 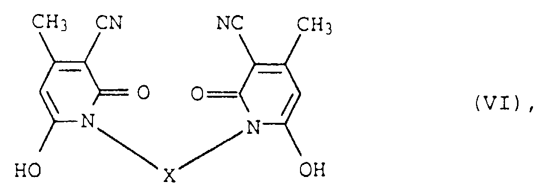 in der X die in Anspruch 8 genannte Bedeutung besitzt, in konzentrierter Schwefelsäure bei einer Temperatur von 80 bis 130°C behandelt.

## Patentansprüche (Patentansprüche für folgende(n) Vertragsstaat(en): ES)

1. Verfahren zur Herstellung der Azofarbstoffe der Formel I 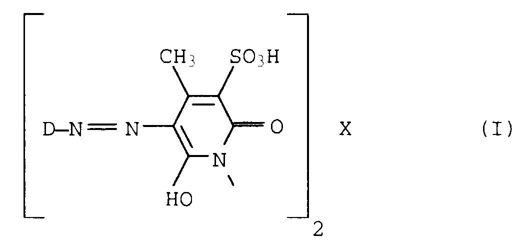 in Form der freien Säure oder ihrer Salze, worin
D den Rest einer Diazokomponente und
X C₂-C₈-Alkylen, das gegebenenfalls durch ein Sauerstoffatom in Etherfunktion unterbrochen ist, bedeuten,
dadurch gekennzeichnet, daß man ein Amin der Formel IV
D-NH₂ (IV),
in der D die obengenannte Bedeutung besitzt, diazotiert und das resultierende Diazoniumsalz mit einem Sulfopyridon der Formal V 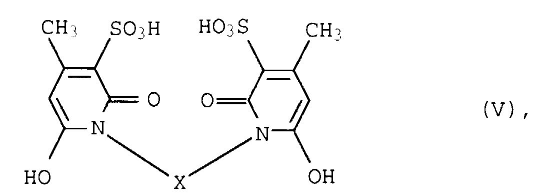 in der X die obengenannte Bedeutung besitzt, kuppelt.

2. Verfahren nach Anspruch 1, dadurch gekennzeichnet, daß D den Rest einer Diazokomponente bedeutet, die sich von einem Anilin, einem Aminonaphthalin oder einem fünfgliedrigen aromatischen heterocyclischen Amin ableitet, das ein bis drei Heteroatome, ausgewählt aus der Gruppe, bestehend aus Stickstoff, Sauerstoff und Schwefel, im heterocyclischen Ring aufweist und durch einen Benzol-, Thiophen-, Pyridin- oder Pyrimidinring anelliert sein kann.

3. Verfahren nach Anspruch 1, dadurch gekennzeichnet, daß D den Rest einer Diazokomponente bedeutet, die sich von einem Anilin, einem Aminonaphthalin oder einem heterocyclischen Amin aus der Pyrrol-, Furan-, Thiophen-, Pyrazol-, Imidazol-, Oxazol-, Isoxazol-, Thiazol-, Isothiazol-, Triazol-, Oxadiazol-, Thiadiazol-, Benzofuran-, Benzthiophen-, Benzimidazol-, Benzoxazol, Benzthiazol-, Benzisothiazol-, Pyridothiophen-, Pyrimidothiophen-, Thienothiophen- oder Thienothiazolreihe ableitet.

4. Verfahren nach Anspruch 1, dadurch gekennzeichnet, daß D den Rest einer Diazokomponente bedeutet, die sich von einem Anilin oder einem Aminonaphthalin ableitet.

5. Verfahren nach Anspruch 1, dadurch gekennzeichnet, daß D den Rest einer Diazokomponente bedeutet, die sich von einem Anilin ableitet.

6. Verfahren nach Anspruch 1, dadurch gekennzeichnet, daß D den Rest einer Diazokomponente bedeutet, die sich von einem Aminobenzophenon, einem Aminoazobenzol oder einer Aminobenzoesäure ableitet.

7. Verwendung der Azofarbstoffe gemäß Anspruch 1 zum Färben oder Bedrucken von natürlichen oder synthetischen Substraten.

8. Verfahren zur Herstellung der Sulfopyridone der Formel V 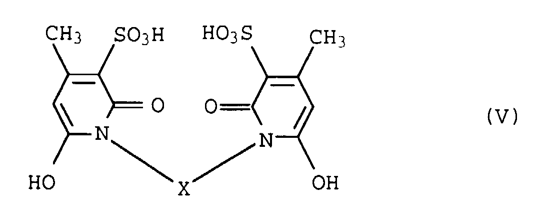 in Form der freien Säure oder ihrer Salze, worin X C₂-C₈-Alkylen, das gegebenenfalls durch ein Sauerstoffatom in Etherfunktion unterbrochen ist, bedeutet, dadurch gekennzeichnet, daß man ein Cyanopyridon der Formel VI 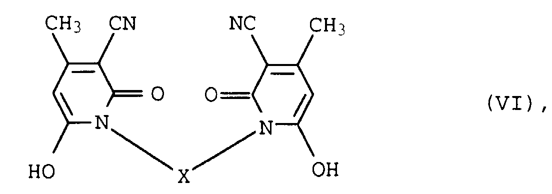 in der X die in Anspruch 8 genannte Bedeutung besitzt, in konzentrierter Schwefelsäure bei einer Temperatur von 80 bis 130°C behandelt.

## Claims (Claims for the following Contracting State(s): CH, DE, FR, GB, IT, LI)

1. Azo dyes of the formula I 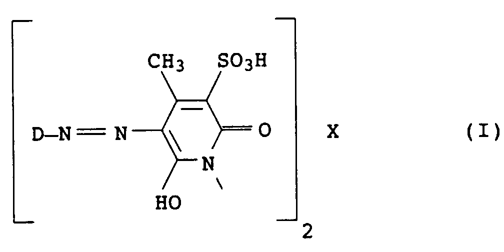 where
D is the radical of a diazo component, and
X is C₂-C₈-alkylene with or without interruption by an oxygen atom in ether function,
in the form of the free acid or its salts.

2. Azo dyes as claimed in claim 1, wherein D is the radical of a diazo component derived from an aniline, from an aminonaphthalene or from a five-membered aromatic heterocyclic amine which contains from one to three hetero atoms selected from the group consisting of nitrogen, oxygen and sulfur in the heterocyclic ring with or without a fused-on benzene, thiophene, pyridine or pyrimidine ring.

3. Azo dyes as claimed in claim 1, wherein D is the radical of a diazo component derived from an aniline, from an aminonaphthalene or from a heterocyclic amine of the pyrrole, furan, thiophene, pyrazole, imidazole, oxazole, isoxazole, thiazole, isothiazole, triazole, oxadiazole, thiadiazole, benzofuran, benzothiophene, benzimidazole, benzoxazole, benzothiazole, benzisothiazole, pyridothiophene, pyrimidothiophene, thienothiophene or thienothiazole series.

4. Azo dyes as claimed in claim 1, wherein D is the radical of a diazo component derived from an aniline or from an aminonaphthalene.

5. Azo dyes as claimed in claim 1, wherein D is the radical of a diazo component derived from an aniline.

6. Azo dyes as claimed in claim 1, wherein D is the radical of a diazo component derived from an aminobenzophenone, from an aminoazobenzene or from an aminobenzoic acid.

7. The use of the azo dyes of claim 1 for dyeing or printing natural or synthetic substrates.

8. Sulfopyridones of the formula V 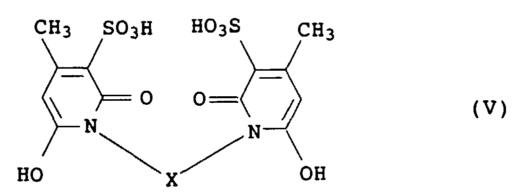 where X is C₂-C₈-alkylene with or without interruption by an oxygen atom in ether function, in the form of the free acid or its salts.

9. A process for preparing the sulfopyridones of claim 8, which comprises treating a cyanopyridone of the formula VI 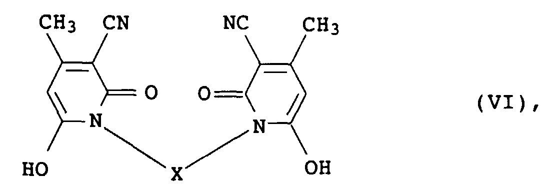 where X is as defined in claim 8, in concentrated sulfuric acid at from 80 to 130°C.

## Claims (Claims for the following Contracting State(s): ES)

1. A process for preparing the azo dyes of the formula I 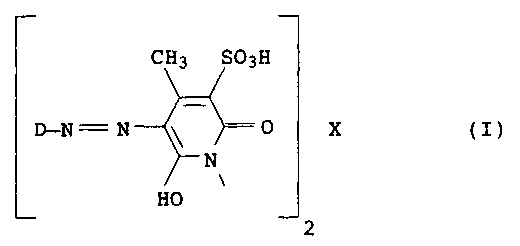 where
D is the radical of a diazo component, and
X is C₂-C₈-alkylene with or without interruption by an oxygen atom in ether function,
in the form of the free acid or its salts,
which comprises diazotizing an amine of the formula IV
D-NH₂ (IV),
where D is as defined above, and coupling the resulting diazonium salt with a sulfopyridone of the formula V 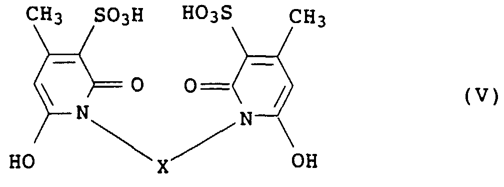 where X is as defined above.

2. A process as claimed in claim 1, wherein D is the radical of a diazo component derived from an aniline, from an aminonaphthalene or from a five-membered aromatic heterocyclic amine which contains from one to three hetero atoms selected from the group consisting of nitrogen, oxygen and sulfur in the heterocyclic ring with or without a fused-on benzene, thiophene, pyridine or pyrimidine ring.

3. A process as claimed in claim 1, wherein D is the radical of a diazo component derived from an aniline, from an aminonaphthalene or from a heterocyclic amine of the pyrrole, furan, thiophene, pyrazole, imidazole, oxazole, isoxazole, thiazole, isothiazole, triazole, oxadiazole, thiadiazole, benzofuran, benzothiophene, benzimidazole, benzoxazole, benzothiazole, benzisothiazole, pyridothiophene, pyrimidothiophene, thienothiophene or thienothiazole series.

4. A process as claimed in claim 1, wherein D is the radical of a diazo component derived from an aniline or from an aminonaphthalene.

5. A process as claimed in claim 1, wherein D is the radical of a diazo component derived from an aniline.

6. A process as claimed in claim 1, wherein D is the radical of a diazo component derived from an aminobenzophenone, from an aminoazobenzene or from an aminobenzoic acid.

7. The use of the azo dyes of claim 1 for dyeing or printing natural or synthetic substrates.

8. A process for preparing the sulfopyridones of the formula V 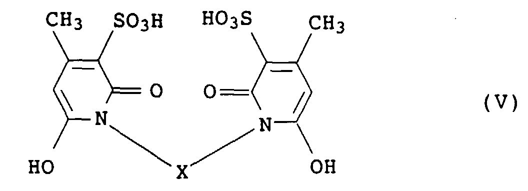 where X is C₂-C₈-alkylene with or without interruption by an oxygen atom in ether function, in the form of the free acid or its salts, which comprises treating a cyanopyridone of the formula VI 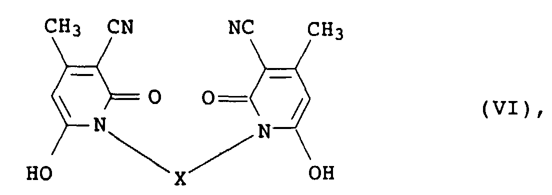 where X is as defined in claim 8, in concentrated sulfuric acid at from 80 to 130°C.

## Revendications (Revendications pour l'(les) Etat(s) contractant(s) suivant(s): CH, DE, FR, GB, IT, LI)

1. Colorants azoïques de formule I 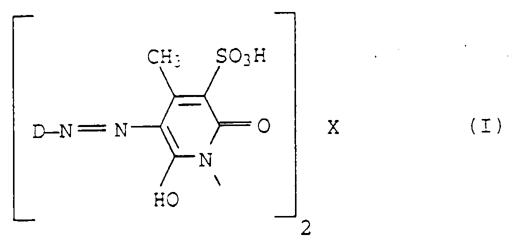 sous forme de l'acide libre ou de ses sels, dans laquelle
D représente le reste d'un composant de diazoïque et
X représente un reste alkylène en C₂-C₈ qui est interrompu par un atome d'oxygène en fonction éther.

2. Colorants azoïques selon la revendication 1, caractérisés en ce que D représente le reste d'un composant de diazoïque qui dérive d'une aniline, d'un aminonaphtalène ou d'une amine hétérocyclique aromatique à cinq chaînons qui comporte, dans le noyau hétérocyclique, 1 à 3 hétéroatomes choisis dans le groupe constitué par l'azote, l'oxygène et le soufre, et qui peut être condensée à un noyau benzène, thiophène, pyridine ou pyrimidine.

3. Colorants azoïques selon la revendication 1, caractérisés en ce que D représente le reste d'un composant de diazoïque qui dérive d'une aniline, d'un aminonaphtalène ou d'une amine hétérocyclique de la série du pyrrole, du furanne, du thiophène, du pyrazole, de l'imidazole, de l'oxazole, de l'isoxazole, du thiazole, de l'isothiazole, du triazole, de l'oxadiazole, du thiadiazole, du benzofuranne, du benzothiophène, du benzimidazole, du benzoxazole, du benzothiazole, du benzisothiazole, du pyridothiophène, du pyrimidothiophène, du thiénothiophène ou du thiénothiazole.

4. Colorants azoïques selon la revendication 1, caractérisés en ce que D représente le reste d'un composant de diazoïque qui dérive d'une aniline ou d'un aminonaphtalène.

5. Colorants azoïques selon la revendication 1, caractérisés en ce que D représente le reste d'un composant de diazoïque qui dérive d'une aniline.

6. Colorants azoïques selon la revendication 1, caractérisés en ce que D représente le reste d'un composant de diazoïque qui dérive d'une aminobenzophénone, d'un aminoazobenzène ou d'un acide aminobenzoïque.

7. Utilisation des colorants azoïques selon la revendication 1 pour la teinture ou l'impression de substrats naturels ou synthétiques.

8. Sulfopyridones de formule V 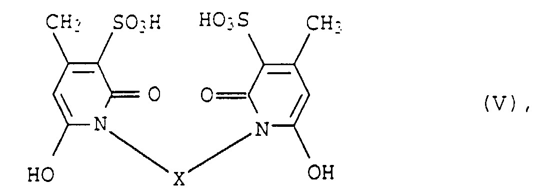 sous forme de l'acide libre ou de ses sels, dans laquelle X représente un reste alkylène en C₂-C₆ qui est éventuellement interrompu par un atome d'oxygène en fonction éther.

9. Procédé de préparation des sulfopyridones selon la revendication 8, caractérisé en ce que l'on traite une cyanopyridone de formule VI 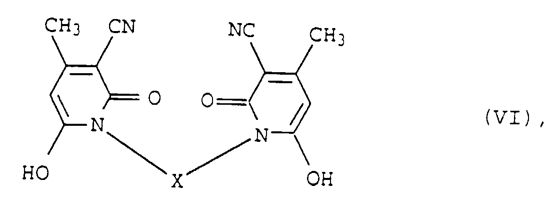 dans laquelle X a la signification donnée dans la revendication 8, dans de l'acide sulfurique concentré à une température de 80 à 130°C.

## Revendications (Revendications pour l'(les) Etat(s) contractant(s) suivant(s): ES)

1. Procédé de préparation des colorants azoïques de formule I 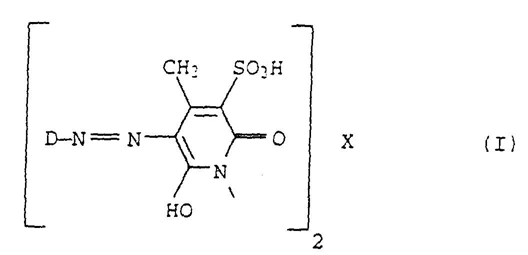 sous forme de l'acide libre ou de ses sels, dans laquelle
D représente le reste d'un composant de diazoïque et
X représente un reste alkylène en C₂-C₈ qui est interrompu par un atome d'oxygène en fonction éther,
caractérisé en ce que l'on diazote une amine de formule IV
D-NH₂ (IV)
dans laquelle D a la signification donnée ci-dessus, et on copule le sel de diazonium résultant avec une sulfopyridone de formule V 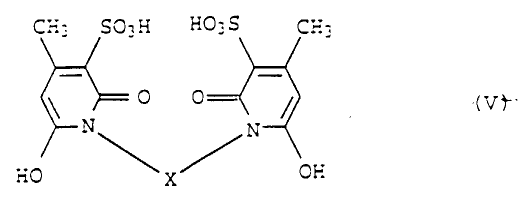 dans laquelle X a la signification donnée ci-dessus.

2. Procédé selon la revendication 1, caractérisé en ce que D représente le reste d'un composant de diazoïque qui dérive d'une aniline, d'un aminonaphtalène ou d'une amine hétérocyclique aromatique à cinq chaînons qui comporte, dans le noyau hétérocyclique, 1 à 3 hétéroatomes choisis dans le groupe constitué par l'azote, l'oxygène et le soufre, et qui peut être condensée à un noyau benzène, thiophène, pyridine ou pyrimidine.

3. Procédé selon la revendication 1, caractérisé en ce que D représente le reste d'un composant de diazoïque qui dérive d'une aniline, d'un aminonaphtalène ou d'une amine hétérocyclique de la série du pyrrole, du furanne, du thiophène, du pyrazole, de l'imidazole, de l'oxazole, de l'isoxazole, du thiazole, de l'isothiazole, du triazole, de l'oxadiazole, du thiadiazole, du benzofuranne, du benzothiophène, du benzimidazole, du benzoxazole, du benzothiazole, du benzisothiazole, du pyridothiophène, du pyrimidothiophène, du thiénothiophène ou du thiénothiazole.

4. Procédé selon la revendication 1, caractérisé en ce que D représente le reste d'un composant de diazoïque qui dérive d'une aniline ou d'un aminonaphtalène.

5. Procédé selon la revendication 1, caractérisé en ce que D représente le reste d'un composant de diazoïque qui dérive d'une aniline.

6. Procédé selon la revendication 1, caractérisé en ce que D représente le reste d'un composant de diazoïque qui dérive d'une aminobenzophénone, d'un aminoazobenzène ou d'un acide aminobenzoïque.

7. Utilisation des colorants azoïques selon la revendication 1 pour la teinture ou l'impression de substrats naturels ou synthétiques.

8. Procédé de préparation des sulfopyridones de formule V, 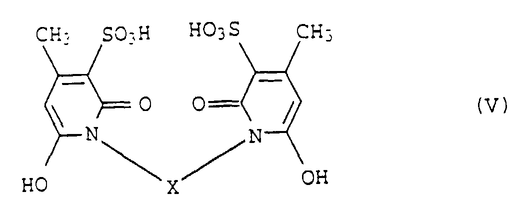 sous forme de l'acide libre ou de ses sels, dans laquelle X représente un reste alkylène en C₂-C₈ qui est éventuellement interrompu par un atome d'oxygène en fonction éther, caractérisé en ce que l'on traite une cyanopyridone de formule VI 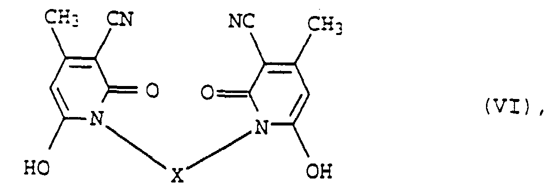 dans laquelle X a la signification donnée dans la revendication 1, dans de l'acide sulfurique concentré à une température de 80 à 130°C.
